# EUROPÄISCHE PATENTSCHRIFT

(11) **EP 0 482 029 B1**
(45) Veröffentlichungstag und Bekanntmachung des Hinweises auf die Patenterteilung: **13.10.1993**
(21) Anmeldenummer: 90910112.3
(22) Anmeldetag: 07.07.1990
(51) Int. Cl.: F16K 7/06, G05D 16/06, A61M 1/00

(54) **SCHLAUCHKLEMME**
HOSE CLAMP
PINCE POUR TUYAU FLEXIBLE

(30) Priorität: 08.07.1989 DE 8908336 U
(43) Veröffentlichungstag der Anmeldung: 29.04.1992
(73) Patentinhaber: MEDINORM AKTIENGESELLSCHAFT MEDIZINTECHNISCHE PRODUKTE, D-66287 Quierschied (DE)
(72) Erfinder: FELL, Helmut Dr. rer. nat., D-6670 St. Ingbert (DE)
(74) Vertreter: Müller, Hans, Dipl.-Ing.
(86) Internationale Anmeldenummer: DE9000507
(87) Internationale Veröffentlichungsnummer: WO9100970

(56) Entgegenhaltungen:
- WO-A-88/05319
- DE-B- 2 356 480
- DE-U- 8 610 275
- FR-A- 2 444 472
- US-A- 4 191 204
- US-A- 4 718 895

## Beschreibung

### TECHNISCHES GEBIET

Die Erfindung betrifft eine Klemme für einen Schlauch, in dessem Inneren ein anderer Druck als in seiner Umgebung herrscht. Derartige Schlauchklemmen sind daher sowohl für Unter- als auch für Überdrucksysteme geeignet. Ein bevorzugtes Einsatzgebiet einer derartigen Schlauchklemme ist die Medizintechnik, wo eine derartige Klemme als Anschlußeinrichtung für eine Unterdruck-Saugflasche zum Absaugen von Wundflüssigkeiten bei der Wunddrainage verwendet werden kann. Eine derartige Saugflasche wird über einen Schlauch mit einem perforierten Wunddrainageschlauch verbunden, der wiederum in eine luftdicht verschlossene Wundhöhle eingebracht wird. Der in der Saugflasche herrschende Unterdruck wirkt somit auch in der Wundhöhle, so daß das dort sich bildende Wundsekret in die Flasche hinein abgesaugt werden kann.

### STAND DER TECHNIK

Zum Verschließen eines Schlauches wie beispielsweise des zwischen der Unterdruck-Saugflasche und dem Wunddrainageschlauch vorhandenen Schlauches, sind sogenannte Schiebeklemmen bekannt (DE-A-37 24 483). Diese Schiebeklemmen besitzen zwei gabelartige Zinken, die V-förmig sich aufweitend aneinander befestigt sind. Durch Verschieben dieser Zinken längs ihrer Längsausdehnung relativ zum Schlauch kann der Schlauchquerschnitt zusammengequetscht und dadurch der Schlauch verschlossen werden. Derartige Schiebeklemmen lassen ein Regulieren, d. h. ein teilweises Versperren des Schlauchquerschnittes praktisch nicht zu.

Oftmals besteht allerdings das Bedürfnis, Schläuche nur teilweise zu versperren. Dieses Bedürfnis tritt beispielsweise bei der eingangs erwähnten Saugflasche in der Medizintechnik auf. Aus wirtschaftlichen Gründen ist ein maximaler Unterdruck in der Saugflasche wünschenswert, damit so möglichst viel Sekret in die Flasche hineingesaugt werden kann. Andererseits wirkt dieser hohe Unterdruck durch den Schlauch hindurch auch auf die Wunde, an der die Flasche angeschlossen ist. Letzteres ist wegen der dadurch bewirkten hohen Saugbelastung im Wundbereich weniger erwünscht. Dieser daraus bewirkte Zielkonflikt wird insoweit gelöst, daß Saugflaschen nicht mit maximalem Unterdruck verwendet werden. Dabei ist zu berücksichtigen, daß die Saugflasche und ihre Schlauchanschlüsse Einwegartikel sind, was bedeutet, daß der wirtschaftliche Aufwand für ihre Herstellung möglichst gering sein muß.

Eine gattungsgemäße Schlauchklemme, mit der ein Schlauchquerschnitt regulierend mehr oder weniger stark versperrt werden kann, ist aus der DE-B-23 56 480 bekannt. Das dort offenbarte Ventil eines Behälters besitzt in seinem Deckelbereich einen Hebel, der sowohl auf einem Gummiballon als auch auf einem Schlauch aufliegt. Dieser Hebel ist außerdem zweifach derartig gelagert, daß er sowohl im Uhrzeigersinn als auch im Gegenuhrzeigersinn verschwenkbar ist. Das Innere des Gummiballons ist mit einem Rohr verbunden, das in das Innere des Behälters hineinragt. Der Schlauch, auf dem der Hebel ebenfalls aufliegt, stellt eine zusätzliche Verbindung des Behälterinnenraums mit der Umgebung des Behälters dar. Beim Zusammenziehen des Gummiballons, d. h. bei einer Volumenverkleinerung von dessem Innenraum, verschwenkt der Hebel beispielsweise im Gegenuhrzeigersinn. Beim Aufblähen des Gummiballons, d. h. beim Vergrößern von dessem Innenraum, verschwenkt der Hebel im Uhrzeigersinn, d. h. in entgegengesetzter Richtung. Bei beiden Schwenkrichtungen wirkt der Hebel auf den Schlauch entlastend, so daß der Schlauchquerschnitt in beiden Fällen vergrößert wird. Nachdem das Innere des Gummiballons über das Rohr mit dem Innenraum des Behälters kommuniziert, wird sich im Gummiballon der Druck einstellen, der im Inneren des Behälters vorhanden ist. Unterschreitet der Innendruck des Behälters einen vorgegebenen Wert, wird sich der Gummiballon zusammenziehen und den Schlauchquerschnitt freigeben. Dadurch wächst der Druck im Inneren des Behälters wieder auf den voreingestellten Wert an. Überschreitet dagegen der Innendruck des Behälters den vorgegebenen Wert, dehnt sich der Gummiballon aus und es wird sich ebenfalls der Schlauchquerschnitt öffnen. Dies hat zur Folge, daß der Überdruck im Ballon abgebaut wird und wieder auf den voreingestellten Wert absinkt. Diese Schlauchklemme dient damit zum Regeln des Innendruckes des Behälters auf einen bestimmten Wert, der vom Außendruck abhängt. Beim Unterschreiten eines bestimmten Innendruckes in dem Behälter wird dieser sich einstellende niedrigere Druck sofort durch Öffnen des Schlauchquerschnittes wieder angehoben. Der Schlauchquerschnitt wirkt damit nicht als Drosselstelle. So kann nicht erreicht werden, daß außerhalb des sich mehr oder weniger stark zusammengequetschten Schlauchquerschnittes ein konstanter Unterdruck aufrecht erhalten werden kann, der weitgehend unabhängig vom im Behälter herrschenden Unterdruck ist.

### DARSTELLUNG DER ERFINDUNG

Ausgehend von diesem Stand der Technik liegt der Erfindung die Aufgabe zugrunde, eine Schlauchklemme der eingangs genannten Art anzugeben, die eine einfache Möglichkeit darstellt, einen Schlauchquerschnitt zu verkleinern, um so an dieser Stelle im Schlauchquerschnitt eine Drosselstelle auszubilden.

Diese Erfindung ist durch die Merkmale des Hauptanspruchs gegeben. Vorteilhafte Weiterbildungen und Ausgestaltungen sind Gegenstand der Unteransprüche.

Die erfindungsgemäße Schlauchklemme für einen an einen Behälter anschließbaren Schlauch, in dessem Inneren relativ zu seiner Umgebung ein anderer Druck vorhanden ist, gemäß dem Oberbegriff des Patentanspruchs 1 zeichnet sich dementsprechend dadurch aus, daß die Verbindung zwischen dem Innenraum der Nebenkammer und dem Schlauch eine nur außerhalb des Behälters vorhandene Nebenleitung oder Öffnung ist, die Nebenleitung oder Öffnung bezogen auf die Klemmstelle auf der dem anzuschließenden Behälter abgewandten Seite des Schlauches angeschlossen ist, die Arme an der Wandung der Nebenkammer so angeordnet sind, daß durch Volumenänderung der Nebenkammer die Arme relativ zueinander bewegbar und dadurch der Schlauch unterschiedlich stark zusammenquetschbar ist, wobei die beiden Arme bei in dem Schlauch anwachsendem Unterdruck sich aufeinander zubewegen und die beiden Arme nur bei in dem Schlauch sich verkleinerndem Unterdruck sich voneinander wegbewegen.

Eine derartige in einer Schlauchverbindung zwischen beispielsweise einer Vakuumsaugflasche und einem Wunddrainageschlauch eingebaute Schlauchklemme kann dadurch selbständig als Drosselventil wirken, da der in dem Schlauch herrschende Druck über die Nebenleitung auch in dem Innenraum der Nebenkammer wirkt und damit bewirkt, daß dieser Innenraum sich relativ zum herrschenden Außendruck entsprechend verstellt. Bei einem in dem Schlauch herrschenden Unterdruck wirkt dieser Unterdruck damit auch in der Nebenkammer, wodurch sich diese zusammenziehen und dadurch der an ihr zumindest eine angeschlossene Arm sich auf den anderen Arm relativ zubewegen kann. Letzteres hat wiederum zur Folge, daß die Arme den Schlauchquerschnitt um ein bestimmtes Maß zusammendrücken werden, Die Schlauchklemme wird den Schlauch so weit zusammendrücken, bis ein Gleichgewicht zwischen dem durch den Schlauchquerschnitt wirkenden Unterdruck und dem Unterdruck in der Nebenkammer entstanden ist. Mit Hilfe einer derartigen Schlauchklemme läßt sich damit eine Vakuumflasche mit maximalem Unterdruck verwenden, ohne daß dieser maximale Unterdruck dann auch in beispielsweise einem Wundbereich herrschen wird.

Gemäß einer ersten vorteilhaften Ausgestaltung dieser Schlauchklemme wird die Nebenkammer durch einen balgartigen Körper gebildet, wobei dieser Körper zwischen den beiden Armen angeordnet ist. Sinvoll ist es dabei, die beiden Arme, zwischen denen der Körper vorhanden ist, an einem Muffenstück verschwenkbar zu befestigen; das Muffenstück kann dabei so ausgebildet sein, daß es über den betreffenden Schlauch, der abgeklemmt werden soll, überziehbar ist. Ferner ist der balgartige Körper mit seinen beiden Endbereichen an den beiden Armen befestigt. Die Drosselwirkung einer derartigen Schlauchklemme hängt von den Materialien und der konstruktiven gegenseitigen Anordnung der verwendeten Einzelbauteile ab.

Um diese baumäßig festgelegte Drosselwirkung zu verändern, kann zwischen den beiden Armen zusätzlich ein elastischer Körper angebracht werden, der als Dämpfungsglied wirkt. Je nachdem, wo dieser elastische Körper angebracht ist, läßt sich die Drosselwirkung verstärken oder abschwächen; Dieser Effekt läßt sich nach einer anderen Ausführungsform der Erfindung auch dadurch erreichen, daß der balgartige Körper längs der Arme an verschiedenen Stellen derselben befestigt werden kann.

Statt des balgartigen Körpers ist es auch möglich, eine Kolben-Zylinder-Anordnung vorzusehen, so daß der Innenraum der Nebenkammer in diesem Fall durch den vom Zylinder, Kolben und der Schlauchwand begrenzten Innenraum gebildet wird. An dem Kolben ist dabei zumindest einer der Arme so befestigt, daß beim Verstellen des Kolbens in dem Zylinder dieser Arm quer zur Längsachse des Schlauches bewegt wird. Der andere gegen den Schlauch anliegende Arm kann in diesem Falle ortsfest vorhanden sein. Der zweite Arm kann auch durch eine den Schlauch umgebende sonstige Konstruktion, wie beispielsweise ein Wandstück, gebildet werden.

Der Kolben wird praktischerweise gegen die Kraft eines elastisch verformbaren Körpers in Richtung auf den Schlauch hinbewegbar sein. Dieser elastisch verformbare Körper kann eine elastisch nachgiebige Feder sein; Es ist allerdings auch möglich, den Schlauch zumindest in seinem von den Armen zusammenquetschbaren Bereich elastisch nachgiebig auszubilden. Sofern der Schlauch ganz zusammengequetscht und damit seine Schlauchwandung in diesem Quetschbereich maximal verformt vorhanden ist, wird sich die in der Schlauchwandung vorhandene Rückstellkraft gegen die drückend anliegende Kolbenkraft richten, so daß bei einer Verringerung des Unterdrukkes in der Nebenkammer die Rückstellkraft des Schlauches den Kolben von der Schlauchwandung weg- und damit zurückbewegen wird.

Weitere Vorteile der Erfindung sind den in den Unteransprüchen ferner angegebenen Merkmalen zu entnehmen.

### KURZE BESCHREIBUNG DER ZEICHNUNG

Die Erfindung wird im folgenden anhand der in der Zeichnung dargestellten Ausführungsbeispiele näher beschrieben und erläutert. Es zeigen:
- Fig. 1: eine perspektivische Ansicht einer Vakuumflasche mit angeschlossenem Verbindungsschlauch, an dem eine erste Ausführungsform der erfindungsgemäßen Schlauchklemme befestigt ist,
- Fig. 2: eine Draufsicht auf die an dem Verbindungsschlauch aufsitzende Schlauchklemme nach Fig. 1,
- Fig. 3: eine Seitenansicht einer zweiten Ausführungsform einer an einer Vakuumflasche angebrachten erfindungsgemäßen Schlauchklemme in ihrer Drosselstellung,
- Fig. 4: eine Seitenansicht der Schlauchklemme nach Fig.3 in teilweiser Schnittdarstellung und
- Fig. 5: einen Längsschnitt durch die erfindungsgemäße Schlauchklemme nach Fig. 3, in verschlossenem Zustand der Saugflasche.

### WEGE ZUR AUSFOHRUNG DER ERFINDUNG

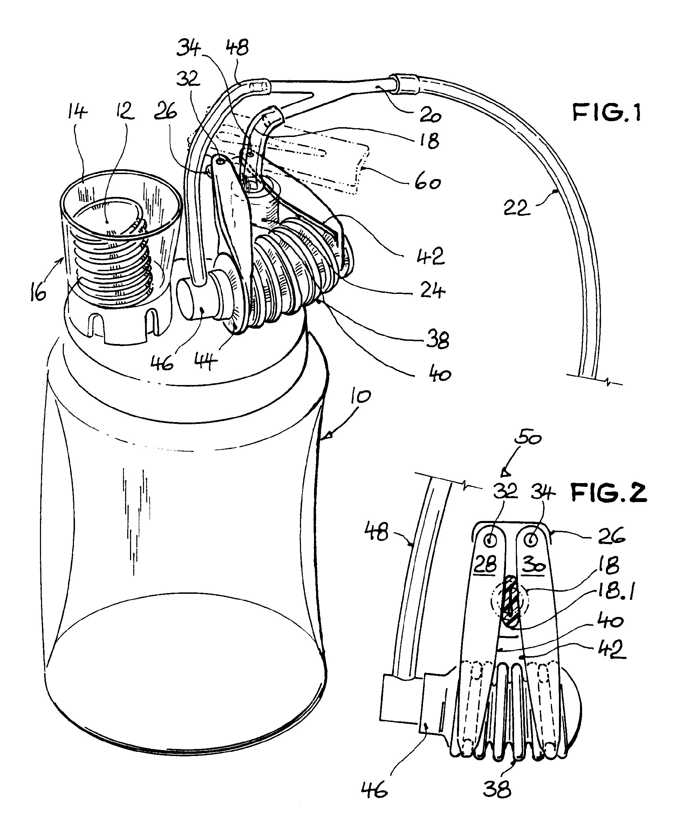

An einer ersten Öffnung einer Vakuum-Saugflasche 10 ist ein Kunststoffbalg 12 angeordnet, der von einem durchsichtigen etwa zylinderförmigen Schutzbecher 14 umgeben ist (Fig. 1, 2). Balg 12 und Schutzbecher 14 wirken als Druckanzeiger 16 für den im Inneren der Saugflasche 10 herrschenden Unterdruck. Ein maximal ausgedehnter Balg 12 zeigt einen minimalen und ein extrem weit zusammengezogener Balg 12 einen maximalen Unterdruck der Saugflasche 10 an.

An einer zweiten Öffnung der Saugflasche 10 ist ein Schlauch 18 angeschlossen. Dieser Schlauch 18 ist über ein Schlauchgabelstück 20 mit einem Schlauch 22 verbunden, der an einen nicht näher dargestellten Drainageschlauch anschließbar ist.

Über den Schlauch 18 ist ein Stutzen 24 übergestülpt. Dieser Stutzen 24 besitzt an seinem von der Saugflasche 10 weggerichteten Ende ein Kragenstück 26. An diesem Kragenstück 26 sind zwei Arme 28, 30 mit ihren Enden mittels jeweils eines Gelenkes 32, 34 schwenkbar befestigt.

Die anderen Enden der beiden Arme 28, 30 sind an einem Balg 38 fest angebracht; Beim Zusammenziehen dieses Balges 38 werden sich die aufeinander zugerichteten Innenseiten 40, 42 der beiden Arme 28, 30 aufeinander zubewegen und den zwischen ihnen vorhandenen Querschnitt des Schlauches 18 mehr oder weniger weit zusammendrücken.

An der - in der Zeichnung linken - Stirnseite 44 des Balges 38 ist über einen Stutzen 46 ein Schlauch 48 mit seinem einen Ende angeschlossen. Das andere Ende dieses Schlauches 48 ist an dem noch freien Stutzen des Schlauchgabelstückes 20 angeschlossen. An den anderen beiden Stutzen dieses Gabelstückes 20 sind der Schlauch 18 und der Schlauch 22 befestigt. Die auf dem Schlauch 18 aufsitzende Schlauchklemme 50 wirkt auf folgende Weise:

Im geöffneten Zustand der Schlauchklemme 50, der in Fig. 1 dargestellt ist, herrscht in dem Schlauch 22 derselbe Unterdruck wie in der Vakuum-Saugflasche 10. Dieser gleiche Unterdruck wirkt nur sehr kurzfristig, da dieser Unterdruck durch den Schlauch 48 hindurch sich auch in dem Balg 38 ausbildet, Der Balg 38 wird s.ich durch diesen Unterdruck aber zusammenziehen, so daß die an ihm angeschlossenen Arme 28, 30 sich zusammenziehen, wie es etwa in Fig. 2 dargestellt ist. Der zwischen den Armen 28 vorhandene, relativ weiche Schlauch wird daher aus seiner strichpunktiert gezeichneten geöffneten Stellung 18 zusammengequetscht, so daß er die in Fig. 2 durchgezogen gezeichnete, zusammengequetschte Stellung 18.1 einnimmt. Auf den bezüglich der Saugflasche 10 weggerichteten Seiten der Arme 28, 30 herrscht damit ein gegenüber dem Inneren der Saugflasche 10 reduzierter Unterdruck. Dieser reduzierte Unterdruck wirkt damit auch in dem Schlauch 22 und damit auch in dem beispielsweise angeschlossenen Wunddrainageschlauch. Trotz maximalem Unterdruck in der Vakuum-Saugflasche 10 wirkt damit ein lediglich reduzierter Unterdruck in einer beispielsweise menschlichen Wunde, aus der Wundsekret in die Vakuum-Saugflasche 10 hinein abgesaugt werden soll,

Um den maximalen Unterdruck vor dem Einsatz der Vakuum-Saugflasche 10 in derselben aufrechtzuerhalten, ist eine strichpunktiert dargestellte Schiebeklemme 60 auf dem Schlauchstück 18 so aufgeschoben, daß der Schlauch 18 an dieser Stelle vollständig verschlossen wird. Der Balg 38 ist damit mit der außerhalb des Balges 38 herrschenden Atmosphäre verbunden, so daß er maximal weit ausgedehnt vorhanden ist. Der Balg 38, der aus Kostenersparnisgründen aus Plastik hergestellt ist, befindet sich damit im entspannten Zustand. Dadurch wird seine elastische Verformbarkeit nicht beeinträchtigt, was im anderen Falle, wo er zusammengedrückt längere Zeit vorhanden wäre, durch Ermüdung des plastischen Materials, aus dem er hergestellt ist, nicht gewährleistet wäre. Sobald die Saugflasche 10 bestimmungsgemäß an beispielsweise einer menschlichen Wunde angeschlossen ist, wird die Schiebeklemme 60 von dem Schlauch 18 entfernt, Dadurch wird sich schlagartig der Balg 38 zusammenziehen und den Schlauch 18 um ein vorbestimmtes Maß zusammenquetschen, so wie es beispielsweise in Fig. 2 dargestellt ist.

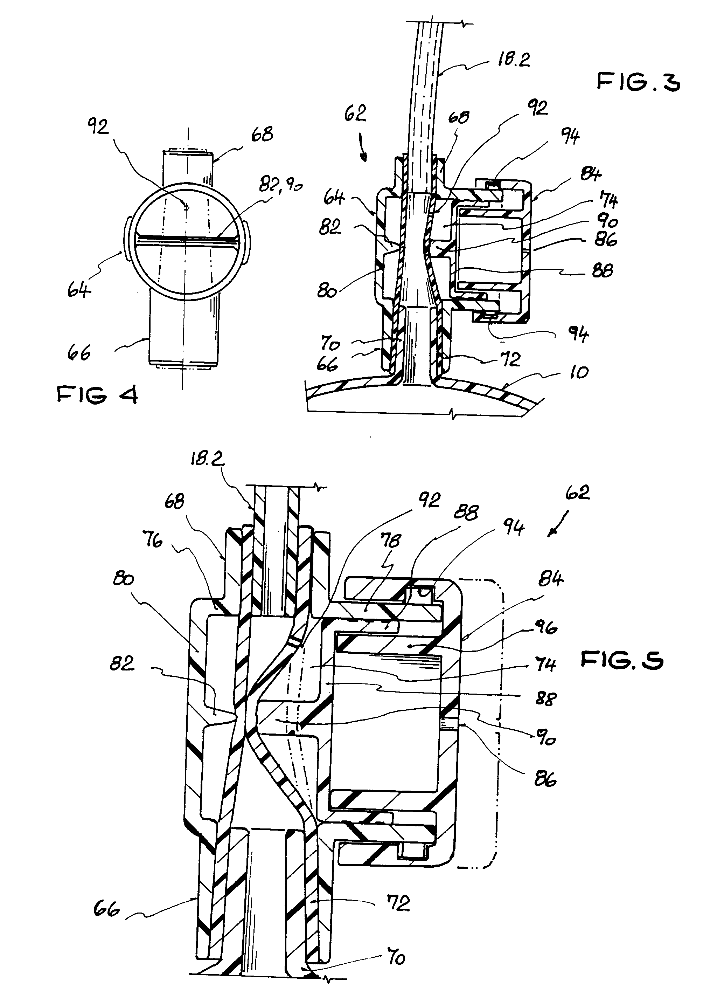

Die in Fig. 3 dargestellte erfindungsgemäße Schlauchklemme 62 besitzt ein Gehäuse 64 mit einem unteren Stutzen 66 und einem oberen Stutzen 68. Der untere Stutzen 66 sitzt gasdicht auf dem Hals 70 der Vakuumflasche 10 auf. Zwischen dem Stutzen 66 und dem Hals 70 ist ein Obergangs-Schlauchstück 72 ebenfalls gasdicht vorhanden. Dieses Schlauchstück 72 läuft durch den Innenraum 74 des Gehäuses 64 hindurch und stößt im Bereich des oberen Stutzens 68 aus dem Gehäuse 64 heraus. Im Bereich des oberen Stutzens 68 ist ein Schlauch 18.2 gasdicht angeschlossen. Das Innere des Schlauches 18.2 ist damit verbunden mit dem Inneren der Vakuumflasche 10.

Beidseitig des Obergangs-Schlauchstückes 72 besitzt das Gehäuse 64 ein linkes Flanschstück 76 sowie ein gegenüberliegendes rechtes Flanschstück 78. An das linke Flanschstück 76 schließt sich eine Wandung 80 an. Diese Wandung besitzt einen mittigen, kragartigen Vorsprung in Form eines linken Armes 82. Nur im Bereich dieses linken Armes 82 liegt die Wand 80 und damit das Gehäuse 64 in diesem Bereich - d. h. in der Zeichnung von links - an dem Obergangs-Schlauchstück 72 an.

Auf der bezüglich der Wand 80 gegenüberliegenden Seite des Gehäuses 64 ist der dortige Flansch 78 von einer hutartigen Kappe 84 bedeckt. In dieser Kappe 84 ist eine Öffnung 86 vorhanden, so daß der außerhalb der Kappe 84 vorhandene atmosphärische Druck auch im Inneren der Kappe 84 wirkt.

In dem Flansch 78 ist - im dargestellten Beispielsfall nach links und rechts - ein Kolben 88 längsverschieblich geführt. Der Kolben 88 liegt dabei gasdicht an der Innenseite des Flansches 78 an. Im vorliegenden Fall besteht dieser Kolben 88 aus Gummi. Dieser Kolben 88 besitzt einen kragartigen Vorsprung in Form eines rechten Armes 90, der bezüglich des Obergangs-Schlauchstückes 72 genau gegenüber dem linken Arm 82 vorhanden ist. Sofern der Kolben 88 ganz nach links verschoben ist, wird das Übergangs-Schlauchstück 72 im Bereich zwischen dem linken Arm 82 und dem rechten Arm 90 extrem weit zusammengequetscht. Dieser Zustand ist in Fig. 5 dargestellt.

In der Wandung des Übergangs-Schlauchstückes 72 ist im Bereich des Gehäuses 64 eine Öffnung 92 vorhanden. Der Druck im Innenraum des übergangs-Schlauchstückes 72 kann sich somit mit dem Druck ausgleichen, der zwischen der Wand 80 und dem Kolben 88 in dem Gehäuse 64 vorhanden ist.

Die Kappe 84 und der Flansch 78 besitzen eine gegenseitige beispielsweise bajonettartige Verrasteinrichtung 94, so daß die Kappe 84 in ihrer in Fig. 5 dargestellten Position festgelegt werden kann. In dieser Stellung der Kappe 64 drückt dieselbe mit angeformten Wandteilen 96 den Kolben 88 extrem weit nach links, so daß das übergangs-Schlauchstück 72 völlig zusammengequetscht ist. Der in der Vakuumflasche herrschende Druck bleibt somit in der Vakuumflasche vorhanden. Die in Fig. 5 dargestellte Stellung der Schlauchklemme 62 ist vor der Benutzung der Saugflasche 10 vorhanden. In dieser Stellung wird die Vakuumflasche 10 mit der an ihr angebrachten Schlauchklemme 62 zu einem Patienten gebracht.

Nachdem der Schlauch 18.2 bzw. der an ihm endseitig angeschlossene Drainageschlauch in der Wunde eines Patienten gasdicht angebracht ist, wird die Kappe 84 entriegelt, d. h., die bajonettartige Verrasteinrichtung 94 wird entriegelt. Die Kappe 84 wird dann etwa ihre in Fig. 3 dargestellte Position einnehmen, was bedeutet, daß der Durchgang durch das übergangs-Schlauchstück 72 mehr oder weniger weit geöffnet vorhanden ist. Der in der Vakuumflasche 10 herrschende Unterdruck wird sich dann durch den Schlauch 18.2 bis in den Wundbereich des Patienten hinein fortsetzen.

Im Bereich zwischen den Armen 82, 90 bildet sich damit ebenfalls eine Drosselstelle aus. Diese Drosselstelle wird beeinflußt durch die Stellung des Kolbens 88. Auf diesen Kolben 88 wirken aus Richtung der Kappe 84 der außerhalb des Gehäuses 94 vorhandene beispielsweise atmosphärische Außendruck, der durch die Öffnung 96 hindurch sich auch in dem Innenraum 74 der Kappe 84 druckmässig einstellen kann. Auf der anderen Seite des Kolbens 88 wirkt der durch den Unterdruck in der Vakuumflasche vorhandene Saugdruck, der regelmäßig niedriger als der atmosphärische Außendruck ist. Dieser Druck kann sich auf der in der Zeichnung linken Seite des Kolbens 88 - im Innenraum 74 - durch das Vorhandensein der Öffnung 92 einstellen. Sobald sich der in dem Innenraum 74 herrschende Druck beispielsweise vergrößert, aber absolut gesehen immer noch kleiner ist als der herrschende Außendruck, wird durch die elastische Ausbildung des Übergangs-Schlauchstückes 72, d. h., durch dessen Rückstellkraft der Kolben 88 nach rechts verschoben. In Abhängigkeit von dem herrschenden Außendruck, dem Druck in der Vakuumflasche und der elastischen Rückstellkraft des Übergangs-Schlauchstückes 72 wird sich damit innerhalb des Gehäuses 64 zwischen den Armen 82 und 90 eine mehr oder weniger stark ausgeprägte Drosselstelle einstellen.

Ebenso wie die Schlauchklemme 50 stellt damit auch die Schlauchklemme 62 eine sehr einfache Möglichkeit zur selbstregulierenden Ausbildung einer Drosselstelle in einem Schlauch dar.

## Patentansprüche

1. Schlauchklemme (50, 62)
für einen an einen Behälter (10) anschließbaren Schlauch (18, 18.2), in dessem Inneren relativ zu seiner Umgebung ein anderer Druck vorhanden ist,
- mit zwei Armen (28, 30, 82, 90), zwischen denen der Schlauch (18, 18.2) unterschiedlich stark zusammenquetschbar und so der Schlauchquerschnitt verschließbar ist,
- wobei die beiden Arme (28, 30, 82, 90) relativ zueinander, quer zur Längsachse des Schlauches (18, 18.2) bewegbar sind und
- wobei eine Nebenkammer (38, 64) vorhanden ist,
-- deren Innenraum eine Verbindung (48, 92) mit dem Schlauch (18, 18.2) besitzt und
-- deren Außenwand zumindest teilweise nachgiebig derart ist, daß das Volumen des Innenraumes veränderbar ist, wobei bei einer volumenmäßigen Veränderung die beiden Arme (28, 30; 82, 90) relativ zueinander bewegbar und so der Schlauch (18, 18.2) unterschiedlich stark zusammenquetschbar ist,
**dadurch gekennzeichnet**, daß
- die Verbindung zwischen dem Innenraum der Nebenkammer (38, 64) und dem Schlauch (18) eine nur außerhalb des Behälters (10) vorhandene Nebenleitung (48) oder Öffnung (92) im Schlauch (18.2) ist,
- die Nebenleitung (48) oder Öffnung (92) bezogen auf die Klemmstelle auf der dem anzuschließenden Behälter (10) abgewandten Seite des Schlauches (18) angeschlossen ist,
- die Arme (28, 30; 82, 90) an der Wandung (44; 80, 88) der Nebenkammer (64) so angeordnet sind, daß durch Volumenänderung der Nebenkammer (64) die Arme (28, 30, 82, 90) relativ zueinander bewegbar und dadurch der Schlauch (18) unterschiedlich stark zusammenquetschbar ist, wobei
-- die beiden Arme (28, 30, 82, 90) bei in dem Schlauch (18) anwachsendem Unterdruck sich aufeinander zubewegen und
-- die beiden Arme (28, 30, 82, 90) nur bei in dem Schlauch (18) sich verkleinerndem Unterdruck sich voneinander wegbewegen.

2. Schlauchklemme (50) nach Anspruch 1,
**dadurch gekennzeichnet**, daß
- die Nebenkammer ein balgartiger Körper (38) ist, der zwischen den Armen (28, 30) angeordnet ist.

3. Schlauchklemme nach Anspruch 2,
**dadurch gekennzeichnet**, daß
- die Arme (28, 30) an einem Muffenstück (24, 26) verschwenkbar (32, 34) befestigt sind,
- dieses Muffenstück über den Schlauch (18) ziehbar ist,
- der balgartige Körper (38) mit seinen beiden Endbereichen an den beiden Armen (28, 30) befestigt ist.

4. Schlauchklemme nach Anspruch 2,
**dadurch gekennzeichnet**, daß
die beiden Arme (28, 30) durch zumindest einen elastischen Körper miteinander verbindbar sind.

5. Schlauchklemme nach Anspruch 2,
**dadurch gekennzeichnet**, daß
der balgartige Körper längs der Arme verstellbar an demselben befestigt ist.

6. Schlauchklemme nach Anspruch 2,
**dadurch gekennzeichnet**, daß
alle ihre Teile aus Kunststoff bestehen.

7. Schlauchklemme nach Anspruch 1,
**dadurch gekennzeichnet**, daß
- ein Zylinder (78) mit einem Kolben (88) vorhanden ist, der Zylinder an einem stirnseitigen Ende geschlossen ist und an dieser Stelle von dem Schlauch (18.2) durchsetzt ist, wobei der Schlauch (18.2) am Zylinder (78) gasdicht befestigt ist, der Zylinder (78) durch die Öffnung (92) im Schlauch (72) mit dem Zylinderinnenraum verbunden ist und das andere stirnseitige Ende des Zylinders (78) durch den in dem Zylinder (78) gasdicht beweglichen Kolben (88) verschlossen ist, wobei der Querschnitt der Öffnung (92) kleiner ist als der Schlauchquerschnitt an der Stelle, an der sich die Öffnung (92) im Schlauch (18.2) befindet,
- der Innenraum (74) der Nebenkammer (64) durch den vom Zylinder (78), vom Kolben (88) und von der Schlauchwand (72) umgebenden Innenraum gebildet wird,
- an der dem Innenraum zugekehrten Wandung des Kolbens (88) zumindest einer der Arme (90) so befestigt ist, daß beim Verstellen des Kolbens (88) dieser Arm (90) quer zur Längsachse des Schlauches bewegbar und dadurch der Schlauch unterschiedlich stark zusammenquetschbar ist.

8. Schlaumklemme nach Anspruch 7,
**dadurch gekennzeichnet**, daß
der Kolben (88) gegen die Kraft eines elastisch verformbaren Körpers in Richtung auf den Schlauch (18.2) hinbewegbar ist.

9. Schlauchklemme nach Anspruch 8,
**dadurch gekennzeichnet**, daß
zumindest der Bereich des Schlauches (18.2) der im Bereich der Arme (82, 90) vorhanden ist, elastisch nachgiebig verformbar ist.

10. Schlauchklemme nach Anspruch 9,
**dadurch gekennzeichnet**, daß
- der Bereich des Schlauches (18.2) zwischen den Armen (82, 90) von einem elastisch nachgiebig verformbaren Übergangs-Schlauchstück (72) gebildet wird,
- an dem einen Ende dieses Schlauchstückes (72) der Schlauch (18.2) und
- an dem anderen Ende dieses Schlauchstückes (72) der Behälter (10) jeweils gasdicht angeschlossen ist.

11. Schlauchklemme nach Anspruch 8,
**dadurch gekennzeichnet,** daß
der elastisch nachgiebige Körper eine Feder ist.

## Claims

1. Flexible tube clip (50, 62) for a flexible tube (18, 18.2) which can be connected to a container (10) and in whose interior tube the pressure prevailing is different from that in its environment,
- having two arms (28, 30, 82, 90) between which the tube (18, 18.2) is compressible to different extents so that the tube cross-section can thus be closed,
- while the two arms (28, 30, 82, 90) are movable relative to one another transversely to the longitudinal axis of the tube (18, 18.2), and
- a subsidiary chamber (38, 64) is provided,
-- the interior of which has a connection (48, 92) to the flexible tube (18, 18.2) and
-- whose outer wall is at least partly resilient so that the volume of the interior space is variable, while on variation of the volume the two arms (28, 30; 82, 90) are movable relative to one another and thus the tube (18, 18.2) can be compressed to different extents, characterised in that
- the connection between the interior space of the subsidiary chamber (38, 64) and the tube (18) is a subsidiary tube (48) or an opening (92) in the flexible tube (18.2), which subsidiary tube or opening is disposed only outside the container (10),
- the subsidiary tube (48) or the opening (92) is connected, in relation to the clamp point, on the side of the tube (18) remote from the container (10) which is to be connected,
- the arms (28, 30; 82, 90) are disposed on the wall (44; 80, 88) of the subsidiary chamber (64) in such a manner that through variation of the volume of the subsidiary chamber (64) the arms (28, 30, 82, 90) are movable relative to one another and the flexible tube (18) can thereby be compressed to different extents, while
-- the two arms (28, 30, 82, 90)
move towards one another when the negative pressure in the flexible tube (18) increases, and
-- the two arms (28, 30, 82, 90)
move away from one another only when the negative pressure in the flexible tube (18) is reduced.

2. Flexible tube clip (50) according to Claim 1, characterised in that
- the subsidiary chamber is a bellows-like body (38) which is disposed between the arms (28, 30).

3. Flexible tube clip according to Claim 2, characterised in that
- the arms (28, 30) are swivellably fastened (32, 34) to a sleeve member (24, 26),
- said sleeve member can be pulled over the tube (18),
- the bellows-like body (38) is fastened by its two end regions to the two arms (28, 30).

4. Flexible tube clip according to Claim 2, characterised in that the two arms (28, 30) can be connected together by at least one elastic body.

5. Flexible tube clip according to Claim 2, characterised in that the bellows-like body is fastened on the arms in such a manner as to be adjustable along them.

6. Flexible tube clip according to Claim 2, characterised in that all its parts are made of plastics material.

7. Flexible tube clip according to Claim 1, characterised in that
- a cylinder (78) is provided which has a piston (88), the cylinder is closed at one end face and at that point the flexible tube (18.2) passes through it, the flexible tube (18.2) being gastightly fastened to the cylinder (78), the cylinder (78) is connected via the opening (92) in the tube (72) to the interior space of the cylinder, and the other end face of the cylinder (78) is closed by the piston (88) gastightly movable in the cylinder (78), while the cross-section of the opening (92) is smaller than the cross-section of the flexible tube at the point at which the opening (92) is situated in the tube (18.2),
- the interior space (74) of the subsidiary chamber (64) is formed by the interior space surrounded by the cylinder (78), the piston (88) and the tube wall (72),
- at least one of the arms (90) is fastened to that wall of the piston (88) which faces the interior space in such a manner that on the displacement of the piston (88) said arm (90) is movable transversely to the longitudinal axis of the tube and the tube is thereby compressible to different extents.

8. Flexible tube clip according to Claim 7, characterised in that the piston (88) can be moved towards the tube (18.2) against the force of an elastically deformable body.

9. Flexible tube clip according to Claim 8, characterised in that at least that region of the tube (18.2) which is situated in the region of the arms (82, 90) is capable of elastically resilient deformation.

10. Flexible tube clip according to Claim 9, characterised in that
- the region of the tube (18.2) between the arms (82, 90) is formed by a transition tubing piece (72) capable of elastically resilient deformation,
- at one end of said tubing piece (72) the tube (18.2) and
- at the other end of this tubing piece (72) the container (10) are connected, gastightly in each case.

11. Flexible tube clip according to Claim 8, characterised in that the elastically resilient body is a spring.

## Revendications

1. Pince à flexible (50, 62) pour un flexible 18, 18.2) pouvant être raccordée à un récipient (10) à l'intérieur duquel existe une autre pression par rapport à son milieu environnant,
- avec deux bras (28, 30, 82, 90) entre lesquels le flexible (18, 18.2) peut être écrasé plus ou moins fortement, si bien que la section du flexible peut être fermée,
- dans laquelle les deux bras (28, 30, 82, 90) peuvent se déplacer relativement l'un vers l'autre, transversalement par rapport à l'axe longitudinal du flexible (18, 18.2), et
- dans lequel il existe une chambre secondaire (38, 64)
-- dont l'enceinte intérieure possède une connexion (48, 92) avec le flexible (18, 18.2) et
-- dont la paroi extérieure est au moins partiellement flexible, de façon que le volume de l'enceinte intérieure puisse être modifié tandis que, en cas de variation de volume, les deux bras (28, 30, 82, 89) peuvent se déplacer relativement l'un par rapport à l'autre et que le flexible (18, 18.2) peut être écrasé plus ou moins fortement,
caractérisée en ce que
- la connexion entre l'enceinte intérieure de la chambre secondaire (38, 64) et le flexible (18) est une conduite secondaire (48) ou une ouverture (92) dans le flexible (18.2) existant uniquement en dehors du récipient (10),
- la conduite secondaire (48) ou l'ouverture (92) est raccordée au côté du flexible (18) opposé au récipient à raccorder (10) par rapport à l'emplacement de la pince,
- les bras (28, 30, 82, 90) sont disposés sur la paroi (44, 80, 88) de la chambre secondaire (64) de façon que, par suite de la variation de volume de la chambre secondaire (64), les bras (28, 30, 82, 90) se déplacent relativement l'un par rapport à l'autre et que le flexible (18) puisse être écrasé plus ou moins fortement tandis que,
-- les deux bras (28, 30, 82, 90) se rapprochent l'un vers l'autre en cas d'augmentation de la dépression dans le flexible (18), et
-- que les deux bras (28, 30, 82, 90) s'écartent l'un de l'autre en cas de diminution de la pression dans le flexible (18).

2. Pince à flexible (50) selon la revendication 1, caractérisée en ce que la chambre secondaire est un corps en forme de soufflet (38) qui est disposé entre les bras (28, 30).

3. Pince à flexible selon la revendication 2, caractérisée en ce que
- les bras (28, 30) sont fixés à pivot (32, 34) à un élément de manchon (24, 26),
- cet élément de manchon peut être tiré par-dessus le flexible (18) et que
- le corps en forme de soufflet (38) est fixé par ses deux zones terminales aux deux bras (28, 30).

4. Pince à flexible selon la revendication 2, caractérisée en ce que les deux bras (28, 30) peuvent être assemblés l'un à l'autre par au moins un corps élastique.

5. Pince à flexible selon la revendication 2, caractérisée en ce que le corps en forme de soufflet est fixé aux bras de manière à pouvoir être déplacé le long de ceux-ci.

6. Pince à flexible selon la revendication 2, caractérisée en ce que toutes ses pièces sont réalisées en matière plastique.

7. Pince à flexible selon la revendication 1, caractérisée en ce que
- il existe un cylindre (78) avec un piston (88), le cylindre étant fermé à l'une de ses extrémités frontales et étant traversé à cet endroit par le flexible (18.2), tandis que le flexible (18.2) est fixé de manière étanche aux gaz au cylindre (78), le cylindre (78) étant connecté par l'ouverture (92) dans le flexible (72) à l'enceinte intérieure du cylindre et l'autre extrémité frontale du cylindre (78) est fermée par le piston (88) pouvant se déplacer de manière étanche aux gaz dans le cylindre (78), tandis que la section de l'ouverture (92) est plus petite que la section du flexible à l'endroit où se trouve l'ouverture (92) dans le flexible (18.2),
- l'enceinte intérieure (74) de la chambre secondaire (64) est formée par le cylindre (78), le piston (88) et l'enceinte intérieure entourée par la paroi de flexible (72),
- sur la paroi du piston (88) tournée vers l'enceinte intérieure est fixé au moins l'un des bras (90), de façon que, en cas de déplacement du piston (88), ce bras (90) puisse se déplacer transversalement par rapport à l'axe longitudinal du flexible et que, de ce fait, le flexible puisse être écrasé plus ou moins fortement.

8. Pince à flexible selon la revendication 7, caractérisée en ce que le piston (88) peut se déplacer en direction du flexible (18.2) contre la force d'un corps élastiquement déformable.

9. Pince à flexible selon la revendication 8, caractérisée en ce qu'au moins la partie du flexible (18.2) qui se trouve dans la région des bras (82, 90) peut être déformée élastiquement.

10. Pince à flexible selon la revendication 9, caractérisée en ce que
- la partie du flexible (18.2) entre les bras (82, 90) est constituée par un élément de flexible de transition (72) élastiquement déformable,
- à l'une des extrémités de cet élément de flexible (72) est raccordé le flexible (18.2) de manière étanche au gaz, et
- à l'autre extrémité de cet élément de flexible (72) est raccordé le récipient (10), également de manière étanche aux gaz.

11. Pince à flexible selon la revendication 8, caractérisé en ce que l'élément élastiquement déformable est un ressort.
